# EUROPEAN PATENT APPLICATION

(11) **EP 3 680 872 A1**
(43) Date of publication of application: **15.07.2020**
(21) Application number: 19150795.3
(22) Date of filing: 08.01.2019
(51) Int. Cl.: G08B 21/04, A61B 5/04, A61B 5/00

(54) **APPARATUS, METHOD AND COMPUTER PROGRAM FOR IDENTIFYING AN OBSESSIVE COMPULSIVE DISORDER EVENT**

(71) Applicant: Nokia Technologies Oy, 02610 Espoo (FI)
(72) Inventor: O'CONNELL, Diarmuid, Co. Kildare (IE)
(74) Representative: Swindell & Pearson Limited

(57) **Abstract**

The application relates to an apparatus, method and computer program for identifying an obsessive compulsive disorder (OCD) event. The event may be identified by monitoring signals obtained from a plurality of biometric sensors wherein the biometric sensors are configured to measure one or more biometric parameters of a subject. When an OCD event is identified one or more outputs maybe provided in response to the identification of the event. The outputs comprise providing instructions to the subject to perform one or more actions to mitigate the OCD event. Further signals are obtained from the plurality of biometric sensors after the one or more instructions have been provided and these signals are used to monitor a response of the subject to the one or more instructions.

## Description

### TECHNOLOGICAL FIELD

Examples of the present disclosure relate to an apparatus, method and computer program for identifying an obsessive compulsive disorder event. Some relate to an apparatus, method and computer program for identifying an obsessive compulsive disorder event using signals obtained from a plurality of biometric sensors.

### BACKGROUND

Obsessive compulsive disorder is a mental health condition in which a person has obsessive thoughts and compulsive behaviors. The thoughts and behaviors associated with the obsessive compulsive disorder may be different for different people.

### BRIEF SUMMARY

According to various, but not necessarily all, examples of the disclosure there may be provided an apparatus comprising means for: obtaining signals from a plurality of biometric sensors wherein the biometric sensors are configured to measure one or more biometric parameters of a subject; monitoring the obtained signals for patterns to identify an obsessive compulsive disorder event; providing, in response to identifying an obsessive compulsive disorder event, one or more outputs wherein the outputs provide instructions to the subject to perform one or more actions to mitigate the obsessive compulsive disorder event; and obtaining further signals from the plurality of biometric sensors after the one or more instructions have been provided and using these signals to monitor a response of the subject to the one or more instructions.

The means may be configured to identify an obsessive compulsive disorder event by comparing the obtained signals from the plurality of biometric sensors to a database of obsessive compulsive disorder events.

The means may be configured to detect an input confirming the obsessive compulsive event and, in response to the detected input update the database of obsessive compulsive disorder events.

The means may be configured to detect an input indicating that the identified event is not an obsessive compulsive event and, in response to the detected input update the database of obsessive compulsive disorder events.

The means may be configured to determine a level of honesty of the detected input.

The means may be configured to use machine learning to monitor the obtained signals for patterns to identify the obsessive compulsive disorder event.

The means may be configured to compare the further signals from the plurality of biometric sensors with an expected response to the one or more instructions and use the comparison to update the outputs provided to the subject.

The plurality of biometric sensors may comprise one or more sensors configured to be worn by the subject.

The plurality of biometric sensors may comprise one or more of: galvanic skin response sensors, heart rate sensors, blood pressure sensors, sweat sensors, accelerometers, gyroscopes, electromyography sensors.

The one or more outputs may be provided by one or more of: one or more displays, one or more speakers, one or more electrodes, one or more heat pads, one or more haptic output devices.

According to various, but not necessarily all, examples of the disclosure there may be provided an apparatus comprising: processing circuitry; and memory circuitry including computer program code, the memory circuitry and the computer program code configured to, with the processing circuitry, cause the apparatus to: obtain signals from a plurality of biometric sensors wherein the biometric sensors are configured to measure one or more biometric parameters of a subject; monitor the obtained signals for patterns to identify an obsessive compulsive disorder event; provide, in response to identifying an obsessive compulsive disorder event, one or more outputs wherein the outputs provide instructions to the subject to perform one or more actions to mitigate the obsessive compulsive disorder event; and obtain further signals from the plurality of biometric sensors after the one or more instructions have been provided and using these signals to monitor a response of the subject to the one or more instructions.

According to various, but not necessarily all, examples of the disclosure there may be provided a method comprising: obtaining signals from a plurality of biometric sensors wherein the biometric sensors are configured to measure one or more biometric parameters of a subject; monitoring the obtained signals for patterns to identify an obsessive compulsive disorder event; providing, in response to identifying an obsessive compulsive disorder event, one or more outputs wherein the outputs provide instructions to the subject to perform one or more actions to mitigate the obsessive compulsive disorder event; and obtaining further signals from the plurality of biometric sensors after the one or more instructions have been provided and using these signals to monitor a response of the subject to the one or more instructions.

The method may comprise identifying an obsessive compulsive disorder event by comparing the obtained signals from the plurality of biometric sensors to a database of obsessive compulsive disorder events.

The method may comprise detecting an input confirming the obsessive compulsive event and, in response to the detected input updating the database of obsessive compulsive disorder events.

The method may comprise detecting an input indicating that the identified event is not an obsessive compulsive event and, in response to the detected input updating the database of obsessive compulsive disorder events.

According to various, but not necessarily all, examples of the disclosure there may be provided a computer program comprising computer program instructions that, when executed by processing circuitry, cause: obtaining signals from a plurality of biometric sensors wherein the biometric sensors are configured to measure one or more biometric parameters of a subject; monitoring the obtained signals for patterns to identify an obsessive compulsive disorder event; providing, in response to identifying an obsessive compulsive disorder event, one or more outputs wherein the outputs provide instructions to the subject to perform one or more actions to mitigate the obsessive compulsive disorder event; and obtaining further signals from the plurality of biometric sensors after the one or more instructions have been provided and using these signals to monitor a response of the subject to the one or more instructions.

The computer program instructions, when executed by processing circuitry may cause identifying an obsessive compulsive disorder event by comparing the obtained signals from the plurality of biometric sensors to a database of obsessive compulsive disorder events.

### BRIEF DESCRIPTION

Some example embodiments will now be described with reference to the accompanying drawings in which:
Fig. 1 shows an example apparatus;
Fig. 2 shows an example system;
Fig. 3 shows an example method;
Fig. 4 shows an example wearable device;
Fig. 5 shows an example pair of wearable devices; and
Figs. 6A to 6C show example methods.

### DETAILED DESCRIPTION

Examples of the present disclosure relate to an apparatus 101, method and computer program for identifying an obsessive compulsive disorder (OCD) event. The event may be identified by monitoring signals obtained from a plurality of biometric sensors 203 wherein the biometric sensors 203 are configured to measure one or more biometric parameters of a subject 401. When an OCD event is identified one or more outputs maybe provided in response to the identification of the event. The outputs comprise providing instructions to the subject 401 to perform one or more actions to mitigate the OCD event. Further signals are obtained from the plurality of biometric sensors 203 after the one or more instructions have been provided and these signals are used to monitor a response of the subject 401 to the one or more instructions.

Examples of the disclosure therefore provide apparatus 101, method and computer programs which enable an OCD event to be identified and also enable a subject 401 to be monitored while they respond to instructions relating to the OCD event. This enables the OCD event to be managed.

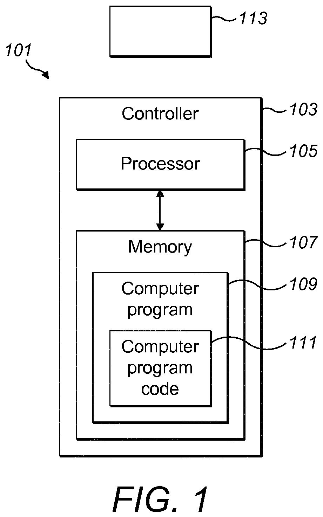

Fig.1 schematically illustrates an apparatus 101 according to examples of the disclosure. In the example of Fig. 1 the apparatus 101 comprises a controller 103. The implementation of the controller 103 may be as controller circuitry. In some examples the controller 103 may be implemented in hardware alone, have certain aspects in software including firmware alone or can be a combination of hardware and software (including firmware).

As illustrated in Fig. 1 the controller 103 may be implemented using instructions that enable hardware functionality, for example, by using executable instructions of a computer program 109 in a general-purpose or special-purpose processor 105 that may be stored on a computer readable storage medium (disk, memory etc) to be executed by such a processor 105.

The processor 105 is configured to read from and write to the memory 107. The processor 105 may also comprise an output interface via which data and/or commands are output by the processor 105 and an input interface via which data and/or commands are input to the processor 105.

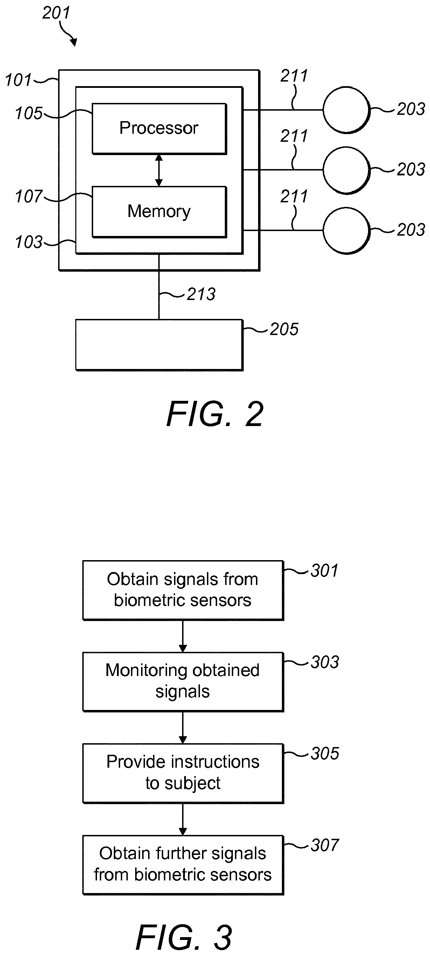

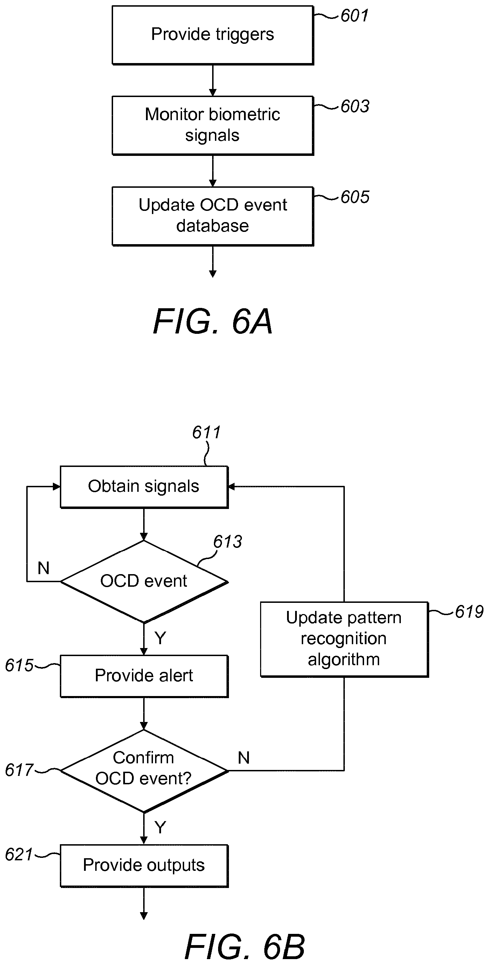

The memory 107 is configured to store a computer program 109 comprising computer program instructions (computer program code 111) that controls the operation of the apparatus 101 when loaded into the processor 105. The computer program instructions, of the computer program 109, provide the logic and routines that enables the apparatus 101 to perform the methods illustrated in Figs. 3 and 6A to 6C. The processor 105 by reading the memory 107 is able to load and execute the computer program 109.

The apparatus 101 therefore comprises: at least one processor 105; and at least one memory 107 including computer program code 111, the at least one memory 107 and the computer program code 111 configured to, with the at least one processor 105, cause the apparatus 101 at least to perform: obtaining 301 signals from a plurality of biometric sensors 203 wherein the biometric sensors 203 are configured to measure one or more biometric parameters of a subject 401; monitoring 303 the obtained signals for patterns to identify an OCD event; providing 305, in response to identifying an OCD event, providing one or more outputs wherein the outputs provide instructions to the subject 401 to perform one or more actions to mitigate the OCD event; and obtaining 307 further signals from the plurality of biometric sensors 203 after the one or more instructions have been provided and using these signals to monitor a response of the subject to the one or more instructions.

As illustrated in Fig.1 the computer program 109 may arrive at the apparatus 101 via any suitable delivery mechanism 113. The delivery mechanism 113 may be, for example, a machine readable medium, a computer-readable medium, a non-transitory computer-readable storage medium, a computer program product, a memory device, a record medium such as a Compact Disc Read-Only Memory (CD-ROM) or a Digital Versatile Disc (DVD) or a solid state memory, an article of manufacture that comprises or tangibly embodies the computer program 109. The delivery mechanism may be a signal configured to reliably transfer the computer program 109. The apparatus 101 may propagate or transmit the computer program 109 as a computer data signal. In some examples the computer program 109 may be transmitted to the apparatus 101 using a wireless protocol such as Bluetooth, Bluetooth Low Energy, Bluetooth Smart, 6LoWPan (IPᵥ6 over low power personal area networks) ZigBee, ANT+, near field communication (NFC), Radio frequency identification, wireless local area network (wireless LAN) or any other suitable protocol.

The computer program 109 comprises computer program instructions for causing an apparatus 101 to perform at least the following: obtaining 301 signals from a plurality of biometric sensors 203 wherein the biometric sensors 203 are configured to measure one or more biometric parameters of a subject 401; monitoring 303 the obtained signals for patterns to identify an OCD event; providing 305, in response to identifying an OCD event, one or more outputs wherein the outputs provide instructions to the subject 401 to perform one or more actions to mitigate the OCD event; and obtaining 307 further signals from the plurality of biometric sensors 203 after the one or more instructions have been provided and using these signals to monitor a response of the subject 401 to the one or more instructions.

The computer program instructions may be comprised in a computer program 109, a non-transitory computer readable medium, a computer program product, a machine readable medium. In some but not necessarily all examples, the computer program instructions may be distributed over more than one computer program 109.

Although the memory 107 is illustrated as a single component/circuitry it may be implemented as one or more separate components/circuitry some or all of which may be integrated/removable and/or may provide permanent/semi-permanent/ dynamic/cached storage.

Although the processor 105 is illustrated as a single component/circuitry it may be implemented as one or more separate components/circuitry some or all of which may be integrated/removable. The processor 105 may be a single core or multi-core processor.

References to "computer-readable storage medium", "computer program product", "tangibly embodied computer program" etc. or a "controller", "computer", "processor" etc. should be understood to encompass not only computers having different architectures such as single /multi- processor architectures and sequential (Von Neumann)/parallel architectures but also specialized circuits such as field-programmable gate arrays (FPGA), application specific circuits (ASIC), signal processing devices and other processing circuitry. References to computer program, instructions, code etc. should be understood to encompass software for a programmable processor or firmware such as, for example, the programmable content of a hardware device whether instructions for a processor, or configuration settings for a fixed-function device, gate array or programmable logic device etc.

As used in this application, the term "circuitry" may refer to one or more or all of the following:
(a) hardware-only circuitry implementations (such as implementations in only analog and/or digital circuitry) and
(b) combinations of hardware circuits and software, such as (as applicable):
   (i) a combination of analog and/or digital hardware circuit(s) with software/firmware and
   (ii) any portions of hardware processor(s) with software (including digital signal processor(s)), software, and memory(ies) that work together to cause an apparatus, such as a mobile phone or server, to perform various functions and
(c) hardware circuit(s) and or processor(s), such as a microprocessor(s) or a portion of a microprocessor(s), that requires software (e.g. firmware) for operation, but the software may not be present when it is not needed for operation.
This definition of circuitry applies to all uses of this term in this application, including in any claims. As a further example, as used in this application, the term circuitry also covers an implementation of merely a hardware circuit or processor and its (or their) accompanying software and/or firmware. The term circuitry also covers, for example and if applicable to the particular claim element, a baseband integrated circuit for a mobile device or a similar integrated circuit in a server, a cellular network device, or other computing or network device.

Fig. 2 shows an example system 201 according to examples of the disclosure. The system 201 comprises an apparatus 101, a plurality of biometric sensors 203 and an output device 205. The apparatus 101 could be as shown in Fig. 1 and corresponding reference numbers are used for corresponding features.

The biometric sensors 203 may comprise any means for detecting biometric parameters from a subject 401. In Fig. 2 the system 201 comprises three biometric sensors 203. Other numbers of biometric sensors 203 could be provided in other examples of the disclosure. The biometric sensors 203 are configured to provide an electrical output signal 211 in response to the biometric parameters.

The biometric parameters that are detected by the biometric sensors 203 may comprise any time varying signal that is generated by the subject's body. In some examples the biometric parameters may comprise one or more autonomic parameters. The autonomic parameters may be controlled subconsciously by the subject 401. The autonomic parameters could comprises temperature, humidity, blood oxygen levels, heart rate, audio signals, pressure, presence of one or more analytes or any other suitable parameters.

In some examples the biometric parameters could comprise one or more parameters that are controlled consciously by the subject. For example the biometric parameters could comprise the movement of the subject 401 and/or part of the subject's 401 body. The movement could comprise the movement of the subject's limbs or torso while performing one or more actions.

In some examples the biometric parameters may comprise one or more bioelectrical signals. The bioelectrical signals comprise electrical signals that are generated within the subject's body. The bioelectrical signals could be generated by the subject's heartbeat, by electrical activity of the subject's brain or other parts of their nervous system or by any other part of the subject's body. In some examples the bioelectric signal could comprise at least one of an electrocardiogram signal, electroencephalogram signal, electromyogram signal, electrooculogram signal, electrogastrogram signal, galvanic skin potential or any other suitable bioelectrical signal.

In some examples the biometric parameters may comprise one or more biomechanical signals. The biomechanical signals could be controlled consciously or subconsciously by the subject. In some examples the biomechanical signals may comprise mechanical signals that are generated within the subject's body by the subject's heartbeat, respiration, abdominal sounds or other body movements. In some examples the biomechanical signal could comprise at least one of ballistocardiogram signal, seismocardiogram signal, phonocardiogram signal or any other suitable signal. In some examples the biomechanical signals could comprise conscious movement of the subject, or part of the subject's body, that could be detected with one or more accelerometers, one or more gyroscopes or any other suitable means.

Other types of biometric parameters could also be detected. For instance, in some examples the biometric sensors 203 could be configured to detect the presence of one or more chemical analytes. This could enable sweat or an increase in sweat to be detected. In some examples the biometric sensors 203 could be configured to detect a temperature of the subject.

In some examples plurality of biometric sensors 203 may be configured so that different sensors detect different types of biometric parameters. For instance a first biometric sensor 203 could detect a bioelectrical signal while a second biometric sensor 203 could detect a biomechanical signal. In some examples the plurality of biometric sensors 203 may be configured so that different sensors 203 detect the same type of biometric parameters but from different locations. For instance a first biometric sensor 203 could detect a biomechanical signal from a first location on a subject's body and a second biometric sensor 203 could detect a biomechanical signal from a second location on a subject's body.

The biometric sensors 203 may be configured so that, when the system 201 is in use, the biometric sensors 203 are positioned in proximity to the subject's body. In some examples one or more of the sensors 203 may be in direct contact with the subject's body. For instance an electrode for detecting galvanic skin response or skin humidity may be in direct contact with the subject's body. In some examples one or more of the biometric sensors 203 could be provided on the subject's body without requiring direct contact with the subject's body. For example an accelerometer or gyroscope could be configured to monitor movement of the subject 401.

The system 201 is configured so that the biometric sensors 203 provide the output signals 211 to the apparatus 101. In some examples at least some of the biometric sensors 203 could be coupled to the apparatus 101 via wired connections which enables the output signals 211 to be provided to the apparatus 101. In some examples at least some of the biometric sensors 203 could be configured to establish a wireless connection with the apparatus 101 to enable the output signals 211 to be transmitted to the apparatus 101 via the wireless connection.

The apparatus 101 may be configured to obtain the output signals from the one or more biometric sensors 203 and process the obtained signals to identify whether or not an OCD event has occurred. The apparatus 101 may also be configured to provide one or more control signals 213 to the output device 205 to enable an output to be provided to a subject when an OCD event is detected.

The output device 205 could comprise any means which enables an output to be provided to the subject 401. The outputs that are provided to the subject 401 comprise one or more instructions to the subject 401 to perform one or more actions to mitigate the detected OCD event. The instructions that are provided may depend on the type of OCD event that has been detected, the subject 401, the biometric parameters that have been detected, the types of output device 205 available and/or any other suitable factor.

In some examples the output device 205 could comprise means for providing an audio output. The means for providing an audio output could comprise one or more loudspeakers or earpieces or any other suitable means. The audio output could comprise spoken instructions. The spoken instructions could be pre-recorded and stored in the memory 107 of the apparatus 101.

In some examples the output device 205 could comprise means for providing a visual output. The means for providing a visual output could comprise one or more displays, a projector for projecting an image or any other suitable means. The visual output could comprise written instructions which may have been stored in the memory 107 of the apparatus 101.

In some examples the output device 205 could comprise means for providing a haptic output. The means for providing a haptic feedback could comprise one or more physical actuators. The physical actuators could comprise components such as motors, electrodes for electrical stimulation of the skin, buzzers, heat pads or any other suitable components. The physical actuators may be configured to provide a soothing or claiming feedback to the subject 401. In some examples the physical actuators could be configured to provide an alert to a subject 401.

It is to be appreciated that the system 201 could comprise additional components that are not shown in Fig. 2. In some examples the system 201 could comprise one or more user input devices. The user input devices could enable a subject 401, or other user, to input information into the system 201. For example this could enable the subject 401, or other user, to make an input confirming whether or not an event has been correctly identified as an OCD event.

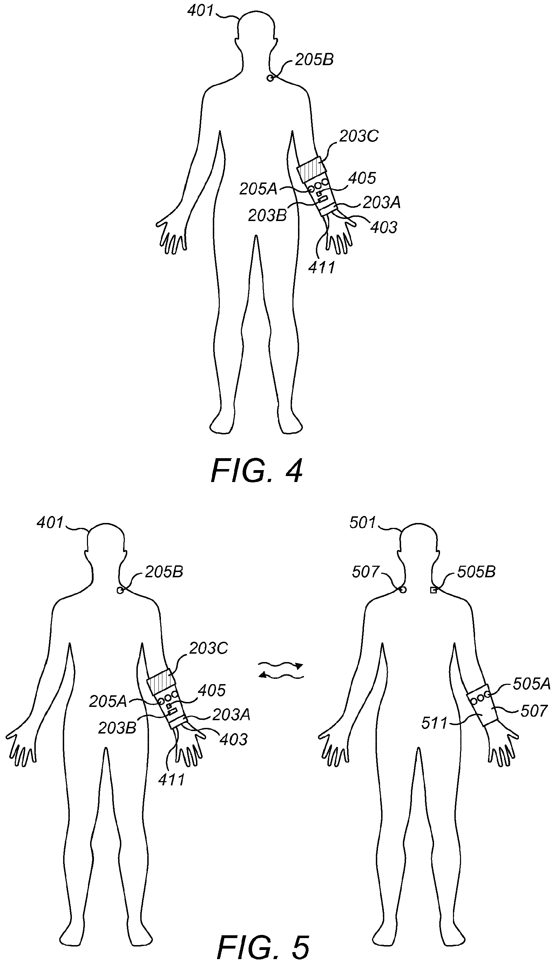

In some examples the system 201 could be provided within a single device. For example a wearable electronic device 403 could be provided which comprises the plurality of biometric sensors 203, an output device 205 and the apparatus 101. Examples of wearable electronic device 403 are shown in Figs. 4 and 5A.

Fig. 3 shows an example method that could be implemented using apparatus 101 and systems 201 as shown in Figs. 1 and 2.

At block 301 the method comprises obtaining signals from a plurality of biometric sensors 203. The biometric sensors 203 are configured to measure one or more biometric parameters of a subject 401. The biometric sensors 203 could be configured to measure a plurality of different types of biometric parameters. In some examples the biometric sensors 203 could be configured to detect the same type of biometric parameter from different locations on a subject's body.

In some examples the biometric sensors 203 could be provided in a wearable device 403 that is worn by the subject 401. This could enable continuous monitoring of the subject 401 or monitoring for extended periods of time.

At block 303 the method comprises monitoring the obtained signals for patterns to identify an OCD event. The monitoring may be performed by the apparatus 101.

An OCD event may comprise any actions or behaviours carried out by the subject 401 that may be indicative of OCD behaviour. Different actions or behaviours could constitute an OCD event in different subjects 401. The OCD event could be a plurality or a sequence of actions and/or biological responses. The actions and/or biological responses could be well defined so that the same, or substantially the same, actions and/or biological parameters are present whenever the OCD event occurs for the subject 401.

In some examples the OCD event could comprise an action such as the subject 401 continuously washing their hands, checking that doors are shut or clenching their hands or performing some other action. In some examples the OCD event could comprise mental processes such as counting steps or feelings of anxiety. In some examples the OCD event could comprise autonomic responses such as elevated heart rate, increase galvanic skin response or changes in other biometric parameters. These actions and/or biological responses can be detected by the biometric sensors 203.

The apparatus 101 is configured to us any suitable process to monitor the obtained signals to enable an OCD event to be identified. In some examples an OCD event may be identified by comparing the obtained signals from the plurality of biometric sensors to a database of OCD events. The database of OCD events could be stored in the memory 107 of the apparatus 101 or could be stored in an external device. In some examples the database could be a predefined database. The predefined database could comprise data that has been obtained from previous test carried out on the subject 401 and/or previous tests carried out on other subjects. In some examples the database could be created via a machine learning process for the subject 401.

In some examples the apparatus 101 may be configured to detect an input confirming whether or not the detected event is an OCD event and, in response to the detected input update the database of OCD events. The input could be the subject 401 or other user actuating a user input device, making an audio input or any other suitable input that could be detected by the apparatus 101.

In some examples the apparatus 101 may be configured to use machine learning to monitor the signals obtained from the biometric sensors 203 for patterns to identify the OCD event. Machine learning is a field of computer science that gives computers the ability to learn without being explicitly programmed. The computer learns from experience E with respect to some class of tasks T and performance measure P if its performance at tasks in T, as measured by P, improves with experience E. The computer can often learn from prior training data to make predictions on future data. Machine learning includes wholly or partially supervised learning and wholly or partially unsupervised learning. It may enable discrete outputs (for example classification, clustering) and continuous outputs (for example regression). The machine learning may be implemented using different approaches such as cost function minimization, artificial neural networks, support vector machines and Bayesian networks for example. Cost function minimization may, for example, be used in linear and polynomial regression and K-means clustering. Artificial neural networks, for example with one or more hidden layers, model complex relationship between input vectors and output vectors. Support vector machines may be used for supervised learning. A Bayesian network is a directed acyclic graph that represents the conditional independence of a number of random variables.

At block 305 the method comprises providing, in response to identifying an OCD event, one or more outputs. The outputs provide instructions to the subject 401 to perform one or more actions to mitigate the OCD event.

The instructions that are to be provided to the subject 401 may be selected so as to mitigate the OCD event. The instructions that are provided may be tailored for the subject 401, the type of OCD event that has been detected or any other suitable factor. The instructions 401 may be stored in the memory 107 of the apparatus 101 and may be retrieved when the OCD event has been detected.

The instructions may comprise instructions for the subject 401 to perform one or more actions to mitigate the OCD event. The actions could include actions such as going for a walk, focussing on a breathing pattern or any other suitable actions. The instructions could comprise a series of actions that are to be performed in a specific sequence.

At block 307 the method comprises obtaining further signals from the plurality of biometric sensors 203 after the one or more instructions have been provided and using these signals to monitor a response of the subject 401 to the one or more instructions. In some examples the further signals from the biometric sensors 203 may be obtained while the subject 401 is receiving the output and/or while the subject 401 is performing an action in accordance with the instructions.

The apparatus 101 may be configured to compare the further signals from the plurality of biometric sensors 203 with an expected response to the one or more instructions provided in the output and use the comparison to update the outputs provided to the subject. The expected response could comprise values for the biometric parameters that have been determined through previous tests on the subject 401. The expected response could be stored in the memory 107 of the apparatus 101.

In some examples the further signals could provide an indication of whether or not the OCD event is being mitigated. For instance it could provide an indication of whether or not the biological parameters are returning to a normal state. The normal state could be based on values of one or more biometric parameters that are specific to a subject 401. The values of the biometric parameters that indicate a normal state could be determined by measurements made by the biometric sensors 203 while the subject 401 is not undergoing an OCD event.

If it is detected that the subject's biometrical parameters have returned to a normal state then the apparatus 101 may control the output device 205 so that no further outputs are provided to the subject 401. If it is detected that the subject's biometrical parameters have not returned to a normal state then the apparatus 101 may control the output device 205 so that further outputs are provided to the subject 401. The further outputs could be further instructions to the subject or a change to the current instructions.

In some examples the further signals obtained from the biometric sensors 203 could provide an indication that the subject 401 is not following the instructions or is not following the instructions correctly. In such examples the apparatus 101 could be configured to provide further outputs to the user. The further outputs could provide a repetition of the current instructions or different instructions.

In some examples the further signals that are obtained from the biometric sensors 203 could be monitored to determine whether or not a different type of OCD event has occurred. For example this could provide an indication that the subject has switched a first type of compulsive behaviour to a second. In response to this the database of OCD events could be updated and additional instructions could be provided to the subject 401.

Fig. 4 shows an example wearable device 403 that may be provided in some examples of the disclosure. The wearable device 403 comprises a plurality of biometric sensors 203. The wearable device 403 could also comprise an apparatus 101 that is coupled to the biometric sensors 203 so that the apparatus 101 can process the output signal 211 from the biometric sensors 203. In some examples the apparatus 101 could be provided as a remote device. For instance the wearable device 403 could comprise one or more transceivers which could be configured to transmit the output signals 211 to the remote device. In such examples the remote device could be a mobile phone or any other suitable processing device.

In the example of Fig. 4 the wearable device 403 comprises a sleeve that is worn around an arm of the subject 401. In the example of Fig. 4 the sleeve 411 is worn around a lower arm of the subject 401.

A plurality of biometric sensors 203 are provided within the sleeve 411. The plurality of biometric sensors 203 are configured to detect different types of biometric signal from the subject 401.

In the example shown in Fig. 4 a first biometric sensor 203A is configured to detect the subject's heart rate. The first biometric sensor 203A could be an infrared sensor configured to detect changes in light absorbed by the subject's blood vessels. Other types of sensor for detecting heart rate could be used in other examples of the disclosure.

A second biometric sensor 203B is configured to detect the subject's galvanic skin response. The galvanic skin response could provide an indication of how much the subject 401 is sweating or other autonomic responses. The second biometric sensor 203B could comprise two or more electrodes which are positioned so as to be in electrical contact with the body of the subject 401.

A third biometric sensor 203C is configured to detect an electromyogram signal. This may provide an indication of muscle activity of the subject 401. This could detect that the subject 401 is moving their arm or is about to move their arm. In some examples this could detect the type of movement of the arm. The third biometric sensor 203C could comprise two or more electrodes which are positioned so as to be in electrical contact with the body of the subject 401. The electrodes may be positioned over the muscles that are of interest for detecting an OCD event. For example, if the subject 401 has a tendency to compulsively wash their hands this would require movement of the muscles in the lower arm which could be detected by electromyography sensors positioned on the lower arm of the subject 401.

It is to be appreciated that the biometric sensors 203 shown in Fig. 4 are examples and that other types of biometric sensors could be used in other examples of the disclosure. Also in the example of Fig. 4 all of the biometric sensors 203 are located on the arm of the subject 401. This could make the wearable device convenient and easy to use. In other examples the biometric sensors 203 could be located in other positions around the body of the subject 401. This could enable different types of biometric signals to be detected.

The biometric sensors 203 that are used may be selected and/or positioned so as to enable particular types of OCD event to be detected. For instance the biometric sensors 203 for detecting electromyogram signals could be positioned in a particular location to detect movement of particular muscles depending on the type of OCD events that are expected for a particular subject 401.

The biometric sensors 203 may also be selected to comprise sensors 203 that can detect actions and biometric parameters that are not associated with OCD events of the subject 401. In some examples this may enable the apparatus 101 to detect that the subject's behavior has returned to normal. In some examples it may enable a new type of OCD event to be identified. For instance, a subject 401 could replace a first type of OCD event with a different type of OCD event. In such examples the apparatus 101 could use machine learning to identify the new type of OCD event.

The wearable device 403 shown in Fig. 4 also comprises a user input device 405. In this examples the user input device 405 comprises a button. The button is located in the wearable device 403 so that it can be actuated by the subject 401. The subject 401 could actuate the button to confirm whether or not a detected event is an OCD event. In some examples the user input device 405 could enable the subject 401 to make a first type of user input to confirm that an event is an OCD event and could make a second type of user input to indicate that a detected event is not an OCD event.

The wearable device 403 also comprises an output device 205. In the example of Fig. 4 the output device 205 comprises means for providing a haptic output. The means for providing haptic output could comprise one or more motors or actuators. These may be configured to provide a force to the subject's skin. This could provide a soothing massaging sensation which may help to mitigate an OCD event. In some examples it could provide an alert to the subject 401 to make them aware of their actions and behaviours.

In the example of Fig. 4 an audio output device 205B is also provided. In this example the audio output device 205B is located on the shoulder of the subject 401 rather than in the sleeve 411 with the biometric sensors 203. The audio output device 205B could be attached to a collar of some clothing of the subject 401. The audio output device 205B is positioned to make it easier for the subject 401 to hear instructions provided via the audio output device 205B. In some examples the audio output device 205B could be coupled to the wearable device 403. For instance, one or more wires could be provided between the wearable device 403 and the audio output device 205B. In other examples the audio output device 205B could be coupled to the apparatus 101 via a wireless connection and need not be directly connected to the wearable device 403.

It is to be appreciated that other types of output device 205 could be provided in other examples of the disclosure. For instance, where the apparatus 101 is provided in a separate device such as a mobile phone, the user interface of the mobile phone could be configured to provide outputs comprising instructions to the subject 401.

It is also to be appreciated that other types of wearable electronic devices 403 could be provided in other examples of the disclosure. For instance in some examples the wearable electronic device 403 could comprise a garment such as a shirt which could comprise one or more biometric sensors 203. In such examples the biometric sensors 203 could be attached to the garment and/or could be embedded within the garment. In some examples the biometric sensors 203 could be provided as one or more sensor pads that could be adhered or otherwise attached to part of the subject's body. The data collected by the sensor pads could then be transmitted to a processing device comprising an apparatus 101 for analysis.

The type of biometric sensors 203 that are provided and the type of wearable electronic device 403 that is used may depend upon the type of actions and behaviours that are associated with an OCD event for the subject 401. A subject 401 who has a compulsion to wash their hands may use a wearable electronic device 401 which comprises biometric sensors 203 that can detect the action of hand washing. This could comprise one or more biometric sensors 203 for detecting an electromyogram signal from muscles that are used when the subject 401 washes their hands or any other suitable type of sensors. A subject 401 who has a compulsion to count steps may use a wearable electronic device 403 which comprises biometric sensors 203 that can detect the action of counting steps. This could comprise motion sensors that detect the subject's steps and a particular cadence or rhythm of the steps and/or any other suitable behaviours or responses.

Fig. 5 shows an example pair of wearable devices 403, 503. The first wearable device 403 is a wearable device for the subject 401 who suffers from OCD and may be as shown in Fig. 4. The second wearable device 503 may be for use by a care giver 501 who may be looking after the subject 401. The care giver 501 could be a medical professional, a relative or any other suitable person.

In the example of Fig. 5 the wearable device 503 for the care giver 501 comprises a sleeve 511 that is worn around an arm of the care giver 501. In the example of Fig. 5 the sleeve 511 is worn around a lower arm of the care giver 501. The wearable device 503 could be worn in other locations in other examples of the disclosure.

The wearable device 503 comprises one or more output devices 505A. In the example of Fig. 5 the output device 505A provided within the sleeve 511 comprises means for providing a haptic output. The means for providing a haptic output could comprise one or more motors or actuators. These could be configured to provide an alert to the care giver 501 if an OCD event is detected for the subject 401.

In the example of Fig. 5 an audio output device 505B is also provided for the care giver 501. This could be one or more speakers that are provided close to the ear of the care giver 501. These could be configured to provide audio information to the care giver 501. The audio information could comprise information about the current condition of the subject 401, any OCD events that have been detected for the subject 401 or any other suitable information.

In the example of Fig. 5 a microphone 507 is also provided for the care giver 501. The microphone enables the care giver 501 to provide audio inputs which could then be transmitted to the subject 401. The microphone 507 may be positioned close to the mouth of the care giver 501 so as to enable the spoken audio inputs to be detected effectively. In some examples the audio inputs that are provided by the care giver 501 could comprise instructions that are to be provided to the subject 401 when an OCD event is detected. These instructions could be provided before the event is detected and could be stored in the memory of the apparatus 107. In some examples these instructions could be provided after an OCD event is detected. For example, the instructions could be provided after the further biometric signals have been obtained. This could enable the care giver 501 to provide updated or adapted instructions to the subject 401.

The wearable device 403 of the subject 401 and the wearable device 503 of the care giver 501 are configured to transmit information between the devices 403, 503. The information may be transmitted via one or more wireless connections. The wireless connection could be direct between the two wearable devices 403, 503. In other examples there could be one or more intervening devices between the two wearable devices 403, 503. For example a processing device, such as a mobile phone, comprising the apparatus 101 could be provided between the two wearable devices 403, 503. The processing device could be configured to obtain the biometric signals from the wearable device 403 of the subject 401 and provide an output to one or both of the wearable devices 403, 503 when an OCD event is detected.

The information that is transmitted from the wearable device 403 of the subject 401 to the wearable device 503 of the care giver 501 could comprise information obtained from the one or more biometric sensors 203. In some examples the information that is transmitted from the wearable device 403 could comprise an indication that the subject 401 is suffering from an OCD event. In some examples the information could comprise a qualitative indication of the OCD event. This could provide the care giver 501 with information about the severity of the OCD event that the subject 401 is suffering. The qualitative indications could be obtained from the information obtained from the one or more biometric sensors 203 and/or from an input from the subject 401 or from any other suitable source. In some example the force and/or delay with which a subject 401 actuates a user input device 405 to confirm that an OCD event is occurring could be used to provide qualitative information about the OCD event.

The information that is transmitted from the wearable device 503 of the care giver 501 to the wearable device 403 of the subject 401 could comprise one or more instructions or other outputs that are to be provided to the subject 401.

Figs. 6A to 6C show example methods that could be implemented using the systems 201, apparatus 101 and wearable devices 403, 503 as described above.

Fig. 6A shows a method that provides a learning phase for examples of the disclosure. The method of Fig. 6A could be performed by an apparatus 101 that could be provided in a wearable device 403 or could be configured to communicate with a wearable device 403. At block 601 the subject 401 is exposed to one or more triggers that may be expected to cause an OCD event. The subject 401 may be connected to one or more biometric sensors 203 while being exposed to the triggers. In some examples the subject 401 could be wearing a wearable device 403 comprising the one or more biometric sensors 203.

The biometric sensors 203 could comprise any suitable types of biometric sensors 203. This could comprise biometric sensors 203 as described above. Different types of biometric sensors 203 could be used to enable different types of biometric signals to be obtained and monitored. Using different types of biometric signals may enable a more accurate database to be created and may reduce the chances of events being incorrectly identified as OCD events.

At block 603 the biometric signals are obtained and monitored to determine the biometric parameters that are associated with the OCD event. At block 605 an OCD event database is updated. In some examples the OCD event database may be created specifically for the subject 401 and may comprise information that is unique to the subject 401. The OCD event database may comprise data which links the biometric parameters to the OCD event that has been triggered.

The method of Fig. 6A may be repeated as many times as appropriate to enable accurate recordings of the biometric signals associated with an OCD event to be determined. In some examples the method may be repeated to enable the database to comprise information relating to different types of OCD event.

In some examples the learning phase could comprise use of a neural scanner such as an fMRI (functional magnetic resonance imaging) scanner, A PET (positron emission tomography) scanner or any other suitable scanner. This may enable the parts of the brain associated with OCD event to be determined.

The method shown in Fig. 6A could be used to set up the apparatus 101 and wearable devices 403 so that the OCD events can be identified for a specific user. The method shown in Fig. 6A could be repeated as many times as needed so as to provide an accurate database of the biometric parameters and the OCD events associated with the parameters. This could also enable the database to be updated if the subject's condition changes and they start having different types of OCD event.

Fig. 6B shows a method that can be used during an OCD event monitoring phase. The method of Fig. 6B could be performed by an apparatus 101 that could be provided in a wearable device 403 or an apparatus 101 that could be configured to communicate with a wearable device 403.

At block 611 a plurality of biometric signals are obtained from the plurality of biometric parameters. At block 613 the obtained biometric signals are monitored to determine whether or not the obtained biometric signals are indicative of an OCD event. The monitoring could comprise comparing the obtained biometric signals with the biometric signals associated with OCD events in the database.

If the obtained biometric signals do not match, or do not substantially match, with the biometric signals listed in the database then no OCD event is identified and the method returns back to block 611 and further biometric signals are obtained. This enables continuous monitoring of the subject 401.

If the obtained biometric signals do match, or substantially match, with the biometric signals listed in the database then an OCD event is identified and at block 615 an alert is provided to the subject 401. The alert could be provided by any of the output devices 205 available to the subject 401. In some examples the alert could be provided via a haptic feedback device such as one or more actuators or motors. This could provide discreet feedback to the subject 401 without disturbing anyone else around the subject 401.

At block 617 it is determined whether or not it is confirmed that the identified event has been correctly identified as an OCD event. The subject 401 could use a user interface to provide a user input which indicates whether or not the event has been correctly identified. For example the subject 401 could actuate a button or other user input device that is provided on the wearable device 403.

If a user input indicating that the event is not an OCD event is detected then, at block 610 the pattern recognition algorithm is updated. This may comprise updating the database to provide more accurate or up to date information about the biometric signals associated with an OCD event. Once the algorithm has been updated the method returns back to block 611 and further biometric signals are obtained so as to enable continuous monitoring of the subject 401.

If a user input indicating that the event is an OCD event is detected, then at block 621, the output devices are controlled to provide outputs to the subject 401. The outputs may comprise one or more instructions to perform one or more actions which may cause the OCD event to be mitigated.

In some examples the apparatus 101 may be configured to determine a level of honesty associated with the input from the subject 401. This could provide an indication of whether or not the subject 401 is honestly accepting whether or not an OCD event is occurring. The level of honesty could be determined from one or more parameters of the way in which the subject 401 actuates the user input device 405. For instance a subject 401 who is actuating the user input device 405 honestly may respond more quickly and apply more force that a subject 401 who is actuating the user input device 405 dishonestly. The parameters that indicate whether or not the subject 401 is actuating the user input device 401 honestly or dishonestly could be determined through tests before the method shown in Fig. 6B is implemented.

The determined level of honesty of the user input could be associated with a weighting factor. The weighting factor gives an indication of whether or not the detected input should be classed as a dishonest input and ignored or if it should be classed as an honest input and used to confirm whether or not an OCD event is occurring.

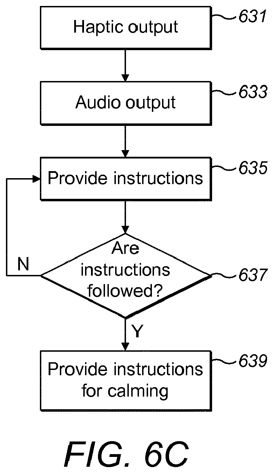

Fig. 6C shows a method that can be used during an output phase. The method of Fig. 6C could be performed by an apparatus 101 that could be provided in a wearable device 403 or could be configured to communicate with a wearable device 403.

At block 631 the output devices 205 are controlled to provide a first output to the subject 401. The first output could comprise a haptic output. The haptic output may be discreet so that people around the subject 401 are not aware that the first output is being provided. The first output may enable the subject to relabel the OCD event.

At block 633 the output devices 205 are controlled to provide a second output to the subject 401. The second output could comprise an audio output. The audio output could be provided via an earpiece or a speaker that is provided close to the subject's ear. The audio output could indicate to the subject 401 that an OCD event has been detected and that they will now be provided with a series of instructions. The audio output may enable reattribution of OCD event.

The audio output could be provided in a voice that is soothing or comforting to the subject. For example the voice could be the voice of a care giver 501 or a relative or other person significant to the subject 401. In some examples the audio output could be pre-recorded and could be retrieved from the memory 107 when the OCD event is detected. In other examples the audio output could be provided via a wearable device 503 from the care giver 501 or other significant person in response to the detection of the OCD event. This audio input could be recorded by a microphone 507 as shown in Fig. 5 and could then be transmitted to the wearable device 403 of the subject 401.

At block 635 the method comprises providing instructions to the subject 401. The instructions may instruct the subject 401 to perform one or more actions that may cause the OCD event to be mitigated. The instructions may comprise instructions to perform one or more actions. The one or more actions may enable refocusing of the subject 401.

The instructions could be provided via any suitable output device 205. For example, the instructions could be provided by audio or visual outputs or by haptic stimulation.

The instructions could instruct the subject 401 to perform one or more actions or activities to mitigate the OCD event. In some examples the actions could be going for a walk. The duration of the walk could be matched to correspond with the typical duration of an OCD event for the subject. Other types of actions could be used in other examples of the disclosure, for instance, the user could be instructed to perform some other types of exercises or follow some breathing patterns.

The instructions could be provided in any suitable format. In some examples audio or written instructions could be provided which may comprise complete instructions for the user. These complete instructions could comprise information about the action that is to be performed and information such as the duration of time for which the action should be performed. In some examples the instructions could comprise prompts which the subject 401 will understand as an instruction to perform an action. For example a haptic feedback device could be configured to provide a haptic output to the subject 401 which the subject 401 has learned to interpret as an instruction to go and perform an action.

At block 637 it is determined whether or not the subject 401 is following the instructions. For instance the biometric signals from the biometric sensors 203 may be monitored to determine whether or not the subject 401 is performing the suggested activity. For example, if the instructions were for the subject 401 to go for a walk for a specified period of time then one or more motion sensors could monitor the movement of the subject to determine if they are walking and how long they have been walking for. Similarly motion sensors and other types of biometric sensors 203 could determine whether or not a specified activity is being performed.

If it is determined that the instructions are not being followed then the method may return to block 63 and further instructions could be provided to the subject 401. In some examples the further instructions could repeat the instructions that have previously been provided to the subject 401. In other examples the further instructions could provide updated or alternative instructions. The updated or alternative instructions could suggest an alternative activity or action.

In some examples further outputs may be provided to the subject 401 while the instructions are being followed. For example if the user is walking or performing some other exercise they could be provided with some audio outputs that help to mitigate the OCD event. The audio output could comprise audio outputs that are prerecorded or otherwise provided from a caregiver 501. In such examples the audio outputs could comprise feedback or encouragement for the subject 401 such as a reminder of how many OCD events have been successfully mitigated. Other types of output that the subject 401 may find calming could be used in other examples of the disclosure.

If it is determined that the instructions are being followed then at block 639 the output devices 205 are controlled to instruct the subject to perform some calming actions such as meditation or controlled breathing exercises. This may enable revaluation for the subject 401. In some examples a haptic output device may be used to help to control a breathing pattern of a subject 401. For instance a stimulus could be provided to help the subject 401 control the timing of their breathing.

The instructions and the outputs that are provided during the output phase may change through repeated use of the apparatus 101. For example, the continued use of the apparatus 101 may lead to an improvement in the subject's symptoms which could reduce the outputs that are required to mitigate an OCD event. Also, if the user changes the type of OCD event this can be addressed by changing the outputs that are provided. In examples of the disclosure the biometric signals are obtained while the outputs are provided so that the subject's response to the outputs can be monitored. This helps to ensure that the condition of the subject 401 can be monitored and an appropriate output can be provided to the subject 401.

It is to be appreciated that different types of outputs and instructions can be provided to the subject 401. The outputs and instructions that are provided may depend on the type of OCD event that is detected, the biometric parameters that are being monitored, personal settings of the subject 401 or any other suitable factor.

Examples of the disclosure provide the benefit that it enables an OCD event to be detected using biometric sensors. This may be beneficial as some OCD events might not be obvious to another person.

The continued monitoring of the subject 401 after the instructions have been provided enables the response of the subject 401 to be monitored. This provides information as to whether or not the OCD event has been mitigated. In some examples this could provide an indication of whether or not the subject 401 has started a different OCD event. This additional information can be used to update the instructions that are provided to the subject 401. The updated instructions could be provided automatically without any further input from the subject 401 or another user.

The term 'comprise' is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to 'comprising only one...' or by using 'consisting'.

In this description, reference has been made to various examples. The description of features or functions in relation to an example indicates that those features or functions are present in that example. The use of the term 'example' or 'for example' or 'can' or 'may' in the text denotes, whether explicitly stated or not, that such features or functions are present in at least the described example, whether described as an example or not, and that they can be, but are not necessarily, present in some of or all other examples. Thus 'example', 'for example', 'can' or 'may' refers to a particular instance in a class of examples. A property of the instance can be a property of only that instance or a property of the class or a property of a subclass of the class that includes some but not all of the instances in the class. It is therefore implicitly disclosed that a feature described with reference to one example but not with reference to another example, can where possible be used in that other example as part of a working combination but does not necessarily have to be used in that other example.

Although embodiments have been described in the preceding paragraphs with reference to various examples, it should be appreciated that modifications to the examples given can be made without departing from the scope of the claims.

Features described in the preceding description may be used in combinations other than the combinations explicitly described above.

Although functions have been described with reference to certain features, those functions may be performable by other features whether described or not.

Although features have been described with reference to certain embodiments, those features may also be present in other embodiments whether described or not.

The term 'a' or 'the' is used in this document with an inclusive not an exclusive meaning. That is any reference to X comprising a/the Y indicates that X may comprise only one Y or may comprise more than one Y unless the context clearly indicates the contrary. If it is intended to use 'a' or 'the' with an exclusive meaning then it will be made clear in the context. In some circumstances the use of 'at least one' or 'one or more' may be used to emphasis an inclusive meaning but the absence of these terms should not be taken to infer and exclusive meaning.

The presence of a feature (or combination of features) in a claim is a reference to that feature or (combination of features) itself and also to features that achieve substantially the same technical effect (equivalent features). The equivalent features include, for example, features that are variants and achieve substantially the same result in substantially the same way. The equivalent features include, for example, features that perform substantially the same function, in substantially the same way to achieve substantially the same result.

In this description, reference has been made to various examples using adjectives or adjectival phrases to describe characteristics of the examples. Such a description of a characteristic in relation to an example indicates that the characteristic is present in some examples exactly as described and is present in other examples substantially as described.

Whilst endeavoring in the foregoing specification to draw attention to those features believed to be of importance it should be understood that the Applicant may seek protection via the claims in respect of any patentable feature or combination of features hereinbefore referred to and/or shown in the drawings whether or not emphasis has been placed thereon.

## Claims

1. An apparatus comprising means for:
obtaining signals from a plurality of biometric sensors wherein the biometric sensors are configured to measure one or more biometric parameters of a subject;
monitoring the obtained signals for patterns to identify an obsessive compulsive disorder event;
providing, in response to identifying an obsessive compulsive disorder event, one or more outputs wherein the outputs provide instructions to the subject to perform one or more actions to mitigate the obsessive compulsive disorder event; and
obtaining further signals from the plurality of biometric sensors after the one or more instructions have been provided and using these signals to monitor a response of the subject to the one or more instructions.

2. An apparatus as claimed in claim 1 wherein the means are configured to identify an obsessive compulsive disorder event by comparing the obtained signals from the plurality of biometric sensors to a database of obsessive compulsive disorder events.

3. An apparatus as claimed in claim 2 wherein the means are configured to detect an input confirming the obsessive compulsive event and, in response to the detected input update the database of obsessive compulsive disorder events.

4. An apparatus as claimed in claim 2 wherein the means are configured to detect an input indicating that the identified event is not an obsessive compulsive event and, in response to the detected input update the database of obsessive compulsive disorder events.

5. An apparatus as claimed in any of claims 3 to 4 wherein the means are configured to determine a level of honesty of the detected input.

6. An apparatus as claimed in any preceding claim wherein the means are configured to use machine learning to monitor the obtained signals for patterns to identify the obsessive compulsive disorder event.

7. An apparatus as claimed in any preceding claim wherein the means are configured to compare the further signals from the plurality of biometric sensors with an expected response to the one or more instructions and use the comparison to update the outputs provided to the subject.

8. An apparatus as claimed in any preceding claim wherein the plurality of biometric sensors comprises one or more sensors configured to be worn by the subject.

9. An apparatus as claimed in any preceding claim wherein the plurality of biometric sensors comprise one or more of: galvanic skin response sensors, heart rate sensors, blood pressure sensors, sweat sensors, accelerometers, gyroscopes, electromyography sensors.

10. An apparatus as claimed in any preceding claim wherein the one or more outputs are provided by one or more of: one or more displays, one or more speakers, one or more electrodes, one or more heat pads, one or more haptic output devices.

11. A method comprising:
obtaining signals from a plurality of biometric sensors wherein the biometric sensors are configured to measure one or more biometric parameters of a subject;
monitoring the obtained signals for patterns to identify an obsessive compulsive disorder event;
providing, in response to identifying an obsessive compulsive disorder event, one or more outputs wherein the outputs provide instructions to the subject to perform one or more actions to mitigate the obsessive compulsive disorder event; and
obtaining further signals from the plurality of biometric sensors after the one or more instructions have been provided and using these signals to monitor a response of the subject to the one or more instructions.

12. A method as claimed in claim 11 comprising identifying an obsessive compulsive disorder event by comparing the obtained signals from the plurality of biometric sensors to a database of obsessive compulsive disorder events.

13. A method as claimed in claim 12 comprising detecting an input confirming the obsessive compulsive event and, in response to the detected input updating the database of obsessive compulsive disorder events.

14. A method as claimed in claim 13 comprising detecting an input indicating that the identified event is not an obsessive compulsive event and, in response to the detected input updating the database of obsessive compulsive disorder events.

15. A computer program comprising computer program instructions that, when executed by processing circuitry, cause:
obtaining signals from a plurality of biometric sensors wherein the biometric sensors are configured to measure one or more biometric parameters of a subject;
monitoring the obtained signals for patterns to identify an obsessive compulsive disorder event;
providing, in response to identifying an obsessive compulsive disorder event, one or more outputs wherein the outputs provide instructions to the subject to perform one or more actions to mitigate the obsessive compulsive disorder event; and
obtaining further signals from the plurality of biometric sensors after the one or more instructions have been provided and using these signals to monitor a response of the subject to the one or more instructions.
